# EUROPÄISCHE PATENTSCHRIFT

(11) **EP 2 592 139 B1**
(45) Veröffentlichungstag und Bekanntmachung des Hinweises auf die Patenterteilung: **24.06.2015**
(21) Anmeldenummer: 12186470.6
(22) Anmeldetag: 25.10.2007
(51) Int. Cl.: C12N 5/07

(54) **Zellkultursystem, Verfahren zu seiner Herstellung sowie seine Verwendung in der präklinischen Untersuchung**
Cell culture system, method for its preparation and its use in pre-clinical examination
Système de culture cellulaire, procédé de fabrication et son utilisation dans l'examen préclinique

(30) Priorität: 25.10.2006 DE 102006051283
(43) Veröffentlichungstag der Anmeldung: 15.05.2013
(62) Teilanmeldung aus: 07819295.2
(73) Patentinhaber: HOT Screen GmbH, 72770 Reutlingen (DE)
(72) Erfinder: Schmolz, Manfred, 72810 Gomaringen (DE)
(74) Vertreter: Gerspacher, Christoph

(56) Entgegenhaltungen:
- LIND M ET AL: "Effects of particulate debris on macrophage-dependent fibroblast stimulation in coculture.", THE JOURNAL OF BONE AND JOINT SURGERY. BRITISH VOLUME SEP 1998, Bd. 80, Nr. 5, September 1998 (1998-09), Seiten 924-930, XP002694711, ISSN: 0301-620X
- HALLER DIRK ET AL: "IL-10 producing CD14low monocytes inhibit lymphocyte-dependent activation of intestinal epithelial cells by commensal bacterian", MICROBIOLOGY AND IMMUNOLOGY, Bd. 46, Nr. 3, 2002, Seiten 195-205, XP002467472, ISSN: 0385-5600
- LIU ET AL: "Studies of intestinal drug transport using an in silico epithelio-mimetic device", BIOSYSTEMS, NORTH-HOLLAND, AMSTERDAM, NL, Bd. 82, Nr. 2, November 2005 (2005-11), Seiten 154-167, XP005135883, ISSN: 0303-2647
- TINTUT YIN ET AL: "Monocyte/macrophage regulation of vascular calcification in vitro", CIRCULATION, Bd. 105, Nr. 5, 5. Februar 2002 (2002-02-05), Seiten 650-655, XP002467473, ISSN: 0009-7322

## Beschreibung

Die vorliegende Erfindung betrifft einen Kit sowie die Verwendung des Kits zur präklinischen Erprobung von Wirkstoffen.

Das präklinische Wirkstoffscreening bzw. die präklinische Erprobung von Wirkstoffen ist von besonderer Bedeutung für die medizinische Validierung von Substanzen, ehe sie nach erfolgreichem Bestehen dieser präklinischen Erprobungsphase in klinischen Studien am Menschen getestet werden. Ein weiterer Schwerpunkt der präklinischen Erprobung bildet neben der Feststellung einer medizinischen Hauptwirkung der zu testenden Substanzen auch die Abschätzung möglicher Nebenwirkungen, die sich bei einer weiteren Wirkstoffvalidierung in klinischen Studien ergeben könnten.

So ermöglicht beispielsweise die Früherkennung von unerwünschten Nebenwirkungen Kosteneinsparungen. Zudem kann das Risiko für klinische Testpatienten durch eine ausgereifte präklinische Wirkstoffuntersuchung deutlich reduziert werden.

Als präklinische Untersuchungsmodelle kommen insbesondere Versuchstiere in Betracht. Tierversuche haben den Vorteil, dass die zu untersuchenden Wirkstoffe in vivo charakterisiert werden können. Allerdings sind die hieraus gewonnenen Erkenntnisse aufgrund der teilweise gravierenden Unterschiede zwischen Tier und Mensch nur in begrenztem Umfang auch auf den Menschen übertragbar.

In der präklinischen Erprobungsphase von Wirkstoffen kommen weiterhin Zellkulturen als Untersuchungsmodelle zum Einsatz. Der Einsatz von Zellkulturen bietet den Vorteil, dass diese relativ einfach durchzuführen sind. Zudem erlauben Zellkulturen hohe Probendurchsatzraten und sehr gut kontrollierbare Testbedingungen. Weiterhin existieren gegen Zellkulturen keine ethischen Bedenken. Nachteilig ist, dass Zellkulturen als in vitro-Untersuchungsmodelle die sich tatsächlich abspielenden zellulären Vorgänge in menschlichen Geweben nur unzureichend simulieren können.

Eine interessante Weiterentwicklung von Zellkulturen stellen die sogenannten Co-Kulturen dar. Solche Co-Kulturen bestehen aus zwei voneinander räumlich separierten Zellkulturen, zwischen denen jedoch ein Stoffaustausch möglich ist. Die eine Zellkultur enthält regelmäßig Zellen bestimmter Gewebetypen, während die andere Zellkultur bestimmte Blutzellen, insbesondere periphere mononukleare Blutzellen (peripheral blood mononuclear cells, PMBC), enthält. Zum präklinischen Testen von Wirkstoffen werden die Zellkulturen in Anwesenheit der zu testenden Wirkstoffe inkubiert. Die in der Co-Kultur ablaufenden Stoffflüsse werden nach der Inkubationssphase untersucht und ausgewertet. Beispielsweise sind derartige Co-Kulturen aus den Artikeln "IL-10 producing CD14low monocytes inhibit lymphocyte-dependent activation of intestinal epithelial cells by commensal bacteria" (Haller D, Microbiol. Immunol. 2002; 46: 195-205) und "Monocyte/Macrophage Regulation of Vascular Calcification In Vitro" (Tintut Y, Circulation 2002, 105: 650-655) bekannt. Nachteilig hierbei ist, dass letztlich auch Co-Kulturen die tatsächlichen Gegebenheiten in Mensch oder Tier nur unzureichend wiedergeben können, insbesondere im Bereich von immunregulativen Vorgängen. Die mit Hilfe solcher Co-Kulturen gewonnenen Erkenntnisse sind daher hinsichtlich einer verlässlichen Beurteilung bzw. Charakterisierung der getesteten Wirkstoffe stets mit einer gewissen Skepsis zu betrachten. Anderseits werden jedoch die Anforderungen an die Qualität eines präklinischen Wirkstoffcreenings wegen der möglichen klinischen Risiken sowie der allgemein immer höher werdenden Entwicklungskosten für Medikamente ständig größer.

Die Erfindung stellt sich somit die Aufgabe, ein in-vitro Untersuchungsmodell zur präklinischen Erprobung von Wirkstoffen bereitzustellen, welches eine gegenüber den aus dem Stand der Technik bekannten Untersuchungsmodellen verbesserte Abbildung der komplexen physiologischen Verhältnisse im menschlichen und/oder tierischen Organismus und insbesondere eine zuverlässigere Charakterisierung der Wirkstoffe mit Hinblick auf ihre klinische Untersuchung ermöglicht.

Diese Aufgabe wird gelöst durch ein Zellkultursystem, insbesondere zur präklinischen Erprobung von Wirkstoffen, umfassend ein erstes und ein zweites Kompartiment, welche über eine für zellulär sezernierte (ausgeschiedene) Stoffe durchlässige Trennschicht zwischen dem ersten und dem zweiten Kompartiment miteinander in Kommunikation stehen, wobei das erste Kompartiment eine syntope Kultur mit Gewebezellen und phagozytierenden Immunzellen und das zweite Kompartiment eine Kultur mit Blutzellen (Blutzellkultur) aufweist.

Unter einer syntopen Kultur im Sinne der vorliegenden Erfindung soll eine Zellkultur verstanden werden, weiche in einem Kompartiment mindestens einen Gewebezelltyp und mindestens einen Zelltyp des Immunsystems aufweist.

Unter Vollblut soll im Sinne der vorliegenden Erfindung Blut mit sämtlichen Blutbestandteilen verstanden werden, einschließlich der Blutzellen und des Blutplasmas sowie der darin enthaltenen vorzugsweise biologisch aktiven Faktoren, wie beispielsweise der Gerinnungsfaktoren, Complementproteine etc.

Unter Priming soll im Sinne der vorliegenden Erfindung eine durch Substanzen, insbesondere durch Botenstoffe, ausgelöste Voraktivierung (Präaktivierung) von Zellen verstanden werden.

Durch die Offenbarung werden Zellkultursysteme bereitgestellt, die sich aufgrund der Berücksichtigung einer syntopen Kultur mit Gewebezellen und phagozytierenden Immunzellen sowie einer Kultur mit Blutzellen in ihrer zellulären Komplexität deutlich von den bisher bekannten Co-Kultursystemen abheben. Das offenbarte Zellkultursystem kann insbesondere als syntope Co-Kultur aufgefasst werden. Im Vergleich zu den bekannten Co-Kultursystemen ermöglicht das offenbarte Zellkultursystem eine wesentlich komplexere und insbesondere differenziertere Kommunikation zwischen den Zellen. Die zwischen den Zellen des Zellkultur systems stattfindenden Stoffflüsse und Regulationsmechanismen erlauben eine deutlich verbesserte Simulation der tatsächlich stattfindenden zellulären Prozesse im menschlichen und/oder tierischen Organismus. Die zellulär ausgeschiedenen Stoffe können insbesondere mit entsprechenden Zielzellen im Zellkultursystem reagieren und können beispielsweise wieder verbraucht werden. Somit können mit besonderem Vorteil unnatürliche Überkonzentrationen im erfindungsgemäßen Zellkultursystem vermieden werden. Weiterhin verändern die Zielzellen ihre eigene Produktion von Signalstoffen unter dem Einfluss der von den anderen Zellen ausgeschiedenen Botenstoffen. Dadurch wird das gesamte regulatorische Netzwerk des erfindungsgemäßen Zellkultursystems auf sehr physiologische Weise modifiziert. Das offenbarte Zellkultursysterm eignet sich in besonderem Maße zur präklinischen Validierung von Wirkstoffen. Hierzu werden zweckmäßigerweise Zellen einer Spezies verwendet, die in einer späteren klinischen Phase mit den zu erprobenden Wirkstoffen behandelt werden soll. Das Zellkultursystem wird zusammen mit den zu erprobendenden Wirkstoffen inkubiert. Die stattfindenden Stoffflüsse und/oder Stoffveränderungen werden vorzugsweise nach der Inkubationsphase erfasst und können insbesondere mit den Stoffflüssen und/oder Stoffveränderungen eines Zellkultursystems verglichen werden, welches ohne Wirkstoffe inkubiert wird. Die hieraus abgeleiten Daten bzw. Informationen können als Grundlage für eine zuverlässige Charakterisierung der untersuchten Wirkstoffe herangezogen werden. So können insbesondere verbesserte Aussagen bezüglich der medizinischen Wirksamkeit der Wirkstoffe sowie bezüglich möglicher Risiken, insbesondere mit Hinblick auf eine nachfolgende klinische Untersuchung, getroffen werden.

In einer bevorzugten Ausführungsform sind die Gewebezellen des ersten Kompartiments adhärent. Vorzugsweise haften die Gewebezellen auf der Oberfläche der Trennschicht. Die Trennschicht kann mit geeigneten Stoffen vorbeschichtet sein. Bei den Stoffen kann es sich beispielsweise um Proteine, insbesondere um extrazelluläre Matrixproteine, handeln. Beispielsweise kann die Trennschicht mit Kollagen, Laminin, Tenascin etc. beschichtet sein.

Die Gewebezellen des ersten Kompartiments sind zweckmäßigerweise auf der Oberfläche der Trennschicht vorgezüchtet. Erfindungsgemäß ist es insbesondere vorgesehen, dass die Gewebezellen die Trennschicht zumindest teilweise, vorzugsweise vollständig, bedecken. Die Gewebezellen können die Trennschicht insbesondere schichtförmig, vorzugsweise als Monoschicht, bedecken.

In einer besonders bevorzugten Ausführungsform handelt es sich bei den phagozytierenden Immunzellen insbesondere um Monozyten und/oder Makrophagen. Die Monozyten und Makrophagen stellen innerhalb des Immunsystems eine immunregulatorische Schaltzentrale dar. Sie sind insbesondere an entzündlichen Prozessen im menschlichen und/oder tierischen Körper beteiligt. Es handelt sich bei der syntopen Kultur des ersten Kompartiments um eine syntope Kultur aus Gewebezellen und phagozytierenden Immunzellen.

Die Immunzellen lagern sich gewöhnlich an die Gewebezellen an, vorzugsweise unter Ausbildung von intermolekularen Adhäsionen. Die Anlagerung der Immunzellen erfolgt insbesondere auf der Basis von an der Zelloberfläche lokalisierten Rezeptoren. Durch die Berücksichtigung der Immunzellen in dem erfindungsgemäßen Zellkultursystem können die in menschlichen und/oder tierischen Körpern ablaufenden entzündlichen Prozesse deutlich besser simuliert werden. Die hieraus abgeleiteten Ergebnisse im Rahmen eines präklinischen Wirkstoffscreenings ermöglichen somit eine zuverlässigere Charakterisierung der mit Hilfe des erfindungsgemäßen Zellkultursystems getesteten Wirkstoffe.

Bei den Gewebezellen der Erfindung handelt es sich vorzugsweise um Zelltypen, welche in entzündlich erkrankenden Geweben, insbesondere in chronisch-entzündlich erkrankenden Geweben, vorkommen. Bei den Geweben kann es sich insbesondere um Organe handeln. Bei den Gewebezellen kann es sich insbesondere um Zellen eines Gewebetyps handeln. In einer weitergehenden Ausführungsform kann die syntope Kultur mehrere Gewebezelltypen enthalten. Dies erhöht in besonderem Maße die Kommunikations- und Regulationsmöglichkeiten zwischen den Zellen des offenbarten Zellkultursystems. Auf diese Weise können die physiologischen Verhältnisse im menschlichen und/oder tierischen Körper, insbesondere auf zellulärer Ebene, in besonders wirkungsvoller Weise simuliert werden.

In einer bevorzugten Ausführungsform handelt es sich bei den Gewebezellen um Epithelzellen und/oder um epithelähnliche Zellen. Besonders bevorzugt handelt es sich bei den Gewebezellen um Epidermis-, Bronchial- und/oder Darmepithelzellen.

In einer weiteren Ausführungsform handelt es sich bei den Gewebezellen um Endothelzellen, bevorzugt um Blutgefäßendothelzellen. Weiterhin ist es bevorzugt, dass es sich bei den Gewebezellen um Hautzellen, insbesondere um Keratinozyten, Fibroblasten und/oder Gelenkhautzellen (sogenannte Synoviozyten), und/oder um Chondrozyten handelt. Weiterhin kommen als Gewebezellen neuronale Zellen und/oder Muskelzellen, insbesondere glatte Muskelzellen, in Frage.

Bevorzugt weist das erste Kompartiment eine syntope Kultur mit Muskelzellen, insbesondere glatten Muskelzellen, und Immunzellen auf, wobei die Muskelzellen vorzugsweise auf der Oberseite der Trennschicht aufgebracht sind. Auf der Unterseite der Trennschicht können insbesondere Endothelzellen aufgebracht sein. Auf diese Weise lassen sich die Verhältnisse von natürlichen Blutgefäßen simulieren.

In einer weitergehenden Ausführungsform stammen die Zellen des erfindungsgemäßen Zellkultursystems, insbesondere die Zellen der syntopen Kultur (Gewebezellen und phagozytierenden Immunzellen), aus Zelllinien. DieZelllinien sind vorzugsweise humanen Ursprungs.

In einer weiteren Ausführungsform stammen die Zellen des offenbarten Zellkultursystems aus Gewebeproben und/oder aus Proben von Körperflüssigkeiten. Bei den Proben kann es sich insbesondere um Primärisolate handeln, d.h. um Proben, welche menschlichen und/oder tierischen Körpern entnommen sind. Die Gewebeproben und/oder die Proben der Körperflüssigkeiten sind vorzugsweise humanen Ursprungs. Bei den Körperflüssigkeiten kann es sich insbesondere um Blut oder Urin, vorzugsweise um Blut, handeln.

Beispielsweise können die Zellen der syntopen Kultur des ersten Kompartiments aus Gewebeproben stammen, wohingegen die Zellen des zweiten Kompartiments aus Blut, stammen.

Es kann weiterhin vorgesehen sein, dass das Zellkultursystem sowohl Zellen aus Zell linien als auch Zellen aus Gewebeproben und/oder aus Proben von Körperflüssigkeiten aufweist. Zweckmäßigerweise enthalten die Kompartimente des offenbarten Zellkultursystems jeweils entweder Zellen aus Zelllinien oder Zellen aus Gewebeproben und/oder aus Proben von Körperflüssigkeiten.

Bei den kultivierten Blutzellen des zweiten Kompartiments kann es sich um Blutzellen eines Blutzelltyps handeln. Vorzugsweise handelt es sich bei den Blutzellen um Zellen des Immunsystems, insbesondere um Zellen des peripheren Blutes. Beispielsweise kann es sich bei den Blutzellen um periphere mononukleare Blutzellen (peripheral blood mononuclear cells, PMBC) handeln. Weiterhin können die Blutzellen des zweiten Kompartiments mehrere Blutzelltypen, insbesondere Lymphozyten, Monozyten, Makrophagen, Thrombozyten und/oder Erythrozyten, umfassen. Vorzugsweise handelt es sich bei der Kultur des zweiten Kompartiments um eine Kultur des Vollblutes (sogenannte Vollblutkultur). In einer Vollblutkultur liegen gewöhnlich alle im natürlichen Blut vorkommenden Blutzellen kultiviert vor.

In einer weitergehenden Ausführungsform ist das Vollblut humanen Ursprungs. Bevorzugt handelt es sich bei dem Vollblut um Frischblut. In einer besonders bevorzugten Ausführungsform weist das Zellkultursystem ausschließlich Zellen humanen Ursprungs auf. Dadurch ist eine verbesserte Simulation der physiologischen Verhältnisse im menschlichen Körper möglich. Die Zellen des Zellkultursystems stammen vorzugsweise aus demselben Organismus, insbesondere aus demselben Patienten.

Die Blutzellkultur des zweiten Kompartiments ist vorzugsweise in Überstand und Sediment getrennt. Im Überstand ist gewöhnlich das Blutplasma enthalten. Im Sediment der Vollblutkultur sind insbesondere die Blutzellen, beispielsweise Erythrozyten, Thrombozyten und Leukozyten, enthalten.

In einer besonders bevorzugten Ausführungsform der Erfindung sind die Gewebezellen aktiviert, vorzugsweise entzündlich verändert. Die Gewebezellen befinden sich insbesondere infolge von im Zellkultursystem anwesenden Aktivatoren, insbesondere proentzündlichen Aktivatoren, in einem aktivierten Zustand. Als Aktivatoren kommen beispielsweise Antigene oder Teile davon, insbesondere Epitope, in Betracht. Bei den Aktivatoren kann es sich auch um Superantige handeln. Die Aktivatoren können mikrobiellen, insbesondere bakteriellen, Ursprungs sein. Vorzugsweise handelt es sich bei den Aktivatoren um Bestandteile von bakteriellen Zellwänden. Bei den Aktivatoren kann es sich insbesondere um Glykane, vorzugsweise um Peptidoglykane, beispielsweise um Zymosan, handeln. Weiterhin kommen Lipopolysaccharide als geeignete Aktivatoren in Frage. Bei den Aktivatoren kann es sich weiterhin um Toxine, beispielsweise Endotoxine, handeln. Durch die Aktivierung der Gewebezellen ist eine Simulation von entzündlichen in vivo Prozessen möglich. Beispielsweise können durch die Aktivierung der Gewebezellen die zellulären Vorgänge bei entzündlich verlaufenden Erkrankungen in zufriedenstellender Weise simuliert werden. Bei den mit Hilfe des offenbarten Zellkultursystems simulierbaren Erkrankungen kann es sich insbesondere um Osteoarthritis, rheumatoide Arthritis, Morbus Crohn, Colitis ulcerosa und entzündliche Lungenerkrankungen handeln.

Bevorzugt sind die Gewebezellen durch Botenstoffe, insbesondere durch glykosylierte Proteine und/oder Peptide, vorzugsweise durch Zytokine, aktiviert. Bei den Botenstoffen handelt es sich bevorzugt um proinflammatorische Mediatoren, beispielsweise um Interferone, Interleukine und/oder Tumornekrosefaktoren (TNF). So können die Gewebezellen insbesondere durch mindestens einen Botenstoff aus der Gruppe, umfassend Interferon y, Interleukin-1, Interleukin-6 und Tumornekrosefaktor α (TNFα), aktiviert sein. Durch die Aktivierung der Gewebezellen können die zellulären Vorgänge in entzündlich erkrankten Geweben, insbesondere in entzündlich erkrankten Organen, wirkungsvoll nachgeahmt werden.

In einer weitergehenden Ausführungsform handelt es sich bei den im offenbarten Zellkultursystem zellulär sezernierten Stoffen allgemein um sogenannte Indikatoren (Indikatorverbindungen) der zellulären Aktivität, insbesondere um Botenstoffe. Vorzugsweise handelt es sich bei den zellulär sezernierten Stoffen um Zytokine. Die zellulär ausgeschiedenen Stoffe können durch die für sie durchlässige Trennschicht in beide Kompartimente des Zellkultursystems diffundieren und insbesondere mit den dort vorhandenen Zellen reagieren. Die Reaktion kann beispielsweise auf Wechselwirkungen der sezernierten Stoffe mit Oberflächenrezeptoren der im jeweiligen Kompartiment vorhandenen Zellen beruhen. Dies reduziert nicht nur das Auftreten von unnatürlichen Konzentrationen, insbesondere von unnatürlichen Überkonzentrationen, von Stoffen im Zellkultursystem. Vielmehr können auf diese Weise auch sekundäre Effekte der zu prüfenden Testsubstanzen überhaupt erst in relevanter Weise sichtbar gemacht werden. Dies ist besonders wichtig für die Untersuchung der Dosis-Wirkungs-Beziehungen der zu testenden Wirkstoffe.

In einer weiteren Ausführungsform handelt es sich bei den Wirkstoffen um natürliche und/oder synthetische Wirkstoffe. Weiterhin kann es sich bei den Wirkstoffen um Wirkstoffmetabolite handeln. Die Metabolite werden beispielsweise durch zellulären Verdau der Wirkstoffe, insbesondere mit Hilfe von Hepatozyten, hergestellt. Die für den zellulären Verdau verwendeten Zellen, insbesondere Hepatozyten, können auch Bestandteil des offenbarten Zellkultursystems sein. Weiterhin können die Wirkstoffe in einer geeigneten Verabreichungsform, beispielsweise zusammen mit einem pharmazeutisch verträglichen Träger, vorliegen. Die Wirkstoffe können beispielsweise Bestandteile von Cremes und/oder Salben sein.

Die für die zellulär sezernierten Stoffe durchlässige Trennschicht zwischen dem ersten und dem zweiten Kompartiments weist zweckmäßigerweise Öffnungen, insbesondere Poren, mit einem Durchmesser zwischen 0.1 und 5 µm, insbesondere zwischen 0,2 und 0,45 µm, auf. Die Trennschicht kann insbesondere Bestandteil des ersten oder des zweiten Kompartiments sein. Vorzugsweise handelt es sich bei der Trennschicht um den Boden des ersten oder des zweiten Kompartiments. Die Trennschicht kann weiterhin mit dem ersten oder dem zweiten Kompartiment, insbesondere mit dem ersten Kompartiment, einstückig ausgebildet sein. Erfindungsgemäß ist es besonders bevorzugt, dass die Trennschicht als Membran oder Diaphragma ausgebildet ist. Die Kompartimente des offenbarten Zellkultursystems können aus verschiedenen Materialien gebildet sein. Als bevorzugte Materialien kommen Kunststoffe, insbesondere Polystyrol oder Polycarbonat, in Frage. Die Kompartimente des offenbarten Zellkultursystems sind vor zugsweise als Behältnisse, insbesondere als Näpfe, Kammern oder Vertiefungen (Wells), ausgebildet. Weiterhin können die Kompartimente Teil eines Trägers sein, welcher vorzugsweise mehrere Kompartimente aufweist. So kann es sich bei dem Träger beispielsweise um eine Lochplatte, insbesondere um eine 6-Loch-, 12-Loch-, 24-Loch- oder 96-Lochplatte, handeln. Bei dem Träger kann es sich weiterhin um ein allgemein kommerziell erhältliches Trans-Well-System für Co-Kulturen handeln.

In einer bevorzugten Ausführungsform handelt es sich bei dem ersten Kompartiment um ein oberes Kompartiment und bei dem zweiten Kompartiment um ein unteres Kompartiment des Zellkultursystems.

Die vorliegende Offenbarung betrifft weiterhin ein Verfahren zur präklinischen Erprobung von Wirkstoffen unter Verwendung eines Zellkultursystems mit einem ersten und einem zweiten Kompartiment, die über eine für zellulär sezernierte (ausgeschiedene) Stoffe durchlässige Trennschicht zwischen dem ersten und dem zweiten Kompartiment miteinander in Kommunikation stehen, wobei das erste Kompartiment eine syntope Kultur mit Gewebezellen und phagozytierende Immunzellen und das zweite Kompartiment eine Kultur mit Blutzellen (Blutzellkultur) aufweist, umfassend die Schritte:
- Zugabe der Wirkstoffe zu dem Zellkultursystem,
- Inkubation des Zellkultursystems in Anwesenheit der zugegebenen Wirkstoffe,
- Untersuchung der im Zellkultursystem nachweisbaren Indikatoren zellulärer Aktivität.

In einer bevorzugten Ausführungsform werden die Wirkstoffe zu der syntopen Kultur des ersten Kompartiments hinzugegeben. In manchen Fällen kann es erwünscht sein, den Einfluss von Wirkstoffen auf Gewebe zu untersuchen, wenn die Wirkstoffe vom Blutkreislauf her in die betreffenden Gewebe gelangen. In diesen Fällen ist es bevorzugt, die Wirkstoffe zu der Blutzellkultur des zweiten Kompartiments hinzuzugeben. Auf diese Weise wird der physiologische Blutkreislauf durch die Blutzellkultur des zweiten Kompartiments und das betreffende Gewebe durch die syntope Kultur des ersten Kompartiments simuliert.

In einer besonders bevorzugten Ausführungsform wird eine syntope Kultur aus Gewebezellen und phagozytierenden Immunzellen, vorzugsweise Monozyten und/oder Makrophagen, verwendet.

In einer weiteren Ausführungsform wird das Zellkultursystem, insbesondere die syntope Kultur, vor der Inkubation, vorzugsweise vor der Zugabe der Wirkstoffe, einem sogenannten Priming unterworfen. In einer anderen Ausführungsform kann das Priming auch nach der Zugabe der Wirkstoffe durchgeführt werden. Es ist auch möglich, die Blutzellkultur des zweiten Kompartiments einem Priming zu unterwerfen. Weiterhin können sowohl die syntope Kultur als auch die Blutzellkultur einem Priming unterworfen werden. Das Priming der syntopen Kultur und der Blutzellkultur kann insbesondere mit jeweils unterschiedlichen Stoffen durchgeführt werden. Als Stoffe für das Priming kommen insbesondere Mediatoren oder Aktivatoren, insbesondere entzündliche Aktivatoren, in Betracht. Bezüglich weiterer Einzelheiten und Merkmale hierzu wird auf die bisherige Beschreibung Bezug genommen.

Das Zellkultursystem wird insbesondere während eines Zeitraums von 1 bis 72 Stunden, vorzugsweise von 2 bis 48 Stunden, inkubiert. Die Inkubation des Zellkultursystems erfolgt zweckmäßigerweise bei einer Temperatur zwischen 20 °C und 40 °C, insbesondere zwischen 35 °C und 40 °C, vorzugsweise bei einer Temperatur von ca. 37 °C. Gegebenenfalls können während der Inkubation des Zellkultursystems weitere Aktivatoren hinzugegeben werden.

In einer weiteren Ausführungsform des offenbarten Verfahrens wird die Untersuchung der im Zellkultursystem nachweisbaren Indikatoren zellulärer Aktivität mittels molekularbiologischer Methoden, insbesondere auf transkriptioneller und/oder translationaler Ebene, vorzugsweise auf post-translationaler Ebene, vorgenommen.

In einer weitergehenden Ausführungsform wird zur Untersuchung der Indikatoren zellulärer Aktivität das Kulturmedium, insbesondere in Form einer Kulturflüssigkeit, des ersten und/oder des zweiten Kompartiments untersucht. Vorzugsweise werden von den Zellen des Zellkultursystems sezernierte Stoffe untersucht. Zur Untersuchung der zellulär sezernierten Stoffe werden insbesondere deren Konzentrationen bestimmt. So können beispielsweise zur Untersuchung der zellulär sezernierten Stoffe ELISA-Verfahren (Enzyme linked immunosorbant assay) oder elektrophoretische Verfahren zum Einsatz kommen. Beispielsweise kann eine Gelelektrophorese, insbesondere eine zweidimensionale Polyacrylamid-Gelelektrophorese (2D PAGE), durchgeführt werden. Weiterhin können Arraytechnologien, insbesondere multiplexe Bead-Arrays, verwendet werden. Grundsätzlich kommen alle dem Fachmann geläufigen biologischen Tests in Frage.

Es ist weiterhin bevorzugt, dass zur Untersuchung der Indikatoren zellulärer Aktivität die Zellen des Zellkultursystems untersucht werden.

Es ist zweckmäßig, dass die Zellen zur Durchführung der Untersuchung zurückgewonnen werden, vorzugsweise nach der Inkubation des Zellkultursystems.

Bevorzugt werden zur Untersuchung der Indikatoren zellulärer Aktivität zellständige Aktivierungsparameter untersucht. Beispielsweise kann der Calcium-Influx in die Zellen des Zellkultursystems gemessen werden. Weiterhin können die Zellen hinsichtlich ihres cAMP/cGAMP-Spiegels (zyklisches Adenosin-Monophosphat/zyklisches Guanidin-Adenosin-Monophosphat-Spiegel) untersucht werden.

Als bevorzugter zellständiger Aktivierungsparameter kommt weiterhin die Expression von Signaltransducern und/oder Rezeptoren in und/oder auf den Zellen des Zellkultursystems in Betracht. Vorzugsweise wird die Dichte der Signaltransducer und/oder Rezeptoren in und/oder auf den Zellen bestimmt. Als geeignete Untersuchungsmethoden kommen insbesondere Oberflächenmarker-Analysen, beispielsweise histologische Färbungen oder Flow-cytometrische Methoden, in Frage.

Weiterhin können zur Untersuchung von zellständigen Aktivierungsparametern die Zellen des Zellkultursystems auf eine Veränderung, insbesondere Aktivierung oder Inhibierung bzw. Suppression, ihrer Gene untersucht werden. Beispielsweise eignen sich zur Profilerstellung von aktivierten Genen Northern-Blot- und/oder Western-Blot-Analysen. Weiterhin kommen planare Arraytechnologien, insbesondere Gen-Chips, in Betracht. Bevorzugt werden die Gene auf der Basis ihrer Genprodukte untersucht. Vorzugsweise wird von den Zellen des Zellkultursystems gebildete RNA (ribonucleic acid), insbesondere messenger RNA (mRNA), untersucht. Die RNA wird zweckmäßigerweise aus den Zellen isoliert und insbesondere gereinigt. Die Isolierung der RNA aus den Zellen kann beispielsweise durch Extraktion erfolgen. Zur weiteren Untersuchung wird die gebildete RNA vorzugsweise einer Amplifikation, insbesondere einer Polymerase-Kettenreaktion vorzugsweise mit reverser Transkriptase (RT-PCR) unterworfen. Der eigentliche Nachweis der RNA wird gewöhnlich mit Hilfe einer Northern-Blot-Technik vorgenommen.

In einer weiteren Ausführungsform werden zur Untersuchung der Indikatoren zellulärer Aktivität von den Zellen des Zellkultursystems exprimierte Proteine untersucht. So können beispielsweise Expressionsmuster bzw. -profile von den Proteinen erstellt werden. Zur Untersuchung der Proteine können insbesondere massenspektroskopische Methoden herangezogen werden. Bei den Proteinen kann es sich insbesondere um Signaltransducer und/oder Rezeptoren handeln. Der Einsatz von Arraytechnologien ist ebenso möglich. Auch die Charakterisierung apoptotischer Signalwege und Vorgänge im hierin beschrieben Zellkultursystem als relevante Endpunkte ist möglich. Die in den vorangegangenen Abschnitten beschriebenen Verfahren sind dem Fachmann hinreichend bekannt, so dass auf eine ausführlichere Darstellung an dieser Stelle verzichtet werden kann.

In einer besonders bevorzugten Ausführungsform des offenbarten Verfahrens werden die Indikatoren zellulärer Aktivität im Verhältnis zu den Indikatoren zellulärer Aktivität eines wirkstofffreien Zellkultursystems, d.h. eines Zellkultursystems, welches ohne Wirkstoffe inkubiert wird, untersucht. Die daraus gewonnenen Daten bzw. Informationen erlauben eine nähere Charakterisierung der untersuchten Wirkstoffe. Bezüglich weiterer Einzelheiten und Merkmale wird auf die bisherige Beschreibung verwiesen.

Die vorliegende Erfindung betrifft einen Kit zur präklinischen Erprobung von Wirkstoffen, welcher ein Kompartiment umfasst welches eine syntope Kultur mit Gewebezellen und phagozytierende Immunzellen aufweist. Bei den phagozytierenden Immunzellen handelt es sich bevorzugt um Monozyten und/oder Makrophagen.

Der erfindungsgemäße Kit umfasst weiterhin ein Kompartiment mit einem Kulturmedium, wobei das Kulturmedium zur Kultivierung von Blutzellen geeignet ist. Das Kulturmedium ist insbesondere eine Kulturflüssigkeit, beispielsweise eine Kulturlösung. Gegebenenfalls kann der erfindungsgemäße Kit ein Blutentnahmebesteck umfassen. Bezüglich weiterer Einzelheiten und Merkmale des Kits wird auf die bisherige Beschreibung verwiesen.

Die vorliegende Offenbarung betrifft außerdem die Verwendung des Zellkultursystems zur präklinischen Erprobung von Wirkstoffen, insbesondere zur Untersuchung von Dosis-Wirkungs-Beziehungen der Wirkstoffe.

Es ist insbesondere vorgesehen, dass mehrere Zellkultursysteme, insbesondere in Form eines Parallelansatzes, für die präklinische Validierung der Wirkstoffe verwendet werden. Dadurch können beispielsweise mehrere Wirkstoffe parallel und insbesondere vergleichend getestet werden. Ebenso ist es möglich, jedes Zellkultursystem einem unterschiedlichen Priming zu unterwerfen. Es kann weiterhin vorgesehen sein, die in einem Parallelansatz verwendeten Zellkultursysteme auf unterschiedliche Indikatoren zellulärer Aktivität zu untersuchen. Auf diese Weise kann insgesamt eine größere Datenmenge bezüglich der zu untersuchenden Wirkstoffe generiert werden, wodurch die Charakterisierung der Wirkstoffe zusätzlich verbessert werden kann. Bezüglich weiterer Einzelheiten und Merkmale wird auf die bisherige Beschreibung Bezug genommen.

Die vorliegende Offenbarung betrifft schließlich die Verwendung einer syntopen Kultur mit Gewebezellen und phagozytierenden Immunzellen zur präklinischen Erprobung von Wirkstoffen, vorzugsweise zur Untersuchung von Dosis-Wirkungs-Beziehungen der Wirkstoffe. Bevorzugt handelt es sich bei den phagozytierenden Immunzellen um Monozyten und/oder Makrophagen. Es handelt sich bei der syntopen Kultur um eine syntope Kultur aus den Gewebezellen und den phagozytierenden Immunzellen.

Weitere Merkmale und Einzelheiten der Erfindung ergeben sich aus den nachfolgenden Figurenbeschreibungen. Dabei können die einzelnen Merkmale jeweils für sich alleine oder zu mehreren in Kombination miteinander verwirklicht sein. Die Figuren werden durch Bezugnahme ausdrücklich zum Inhalt der Beschreibung gemacht.

In den Figuren ist schematisch wiedergegeben:
- Figur 1:: Zellkultursystem,
- Figur 2:: Botenstoff bzw. Mediatorensynthese in klassischen Co-Kultursystemen und einem erfindungsgemäßen Zellkultursystem,
- Figur 3:: Einfluss von Diclofenac auf die Botenstoff bzw. Mediatorensynthese in klassischen Co-Kultursystemen und einem erfindungsgemäßen Zellkultursystem.

### Figurenbeschreibung

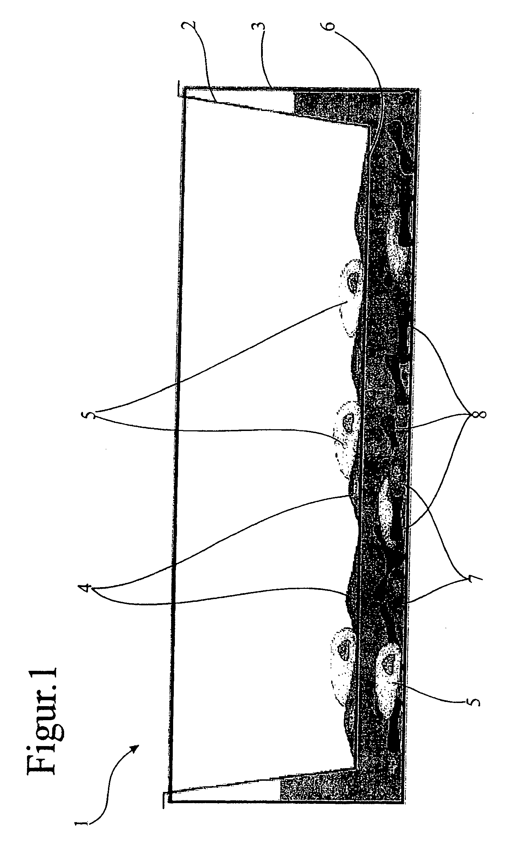

Die Figur 1 zeigt ein Zellkultursystem 1, welches aus einem als erstes Kompartiment ausgebildeten oberen Behältnis 2 und einem als zweites Kompartiment ausgebildeten unteren Behältnis 3 besteht. Das obere Behältnis 2 weist eine syntope Kultur aus Synoviozyten 4 und Monozyten bzw. Makrophagen 5 auf. Die Synoviozyten 4 sind adhärent und bedecken den als Trennschicht ausgebildeten Boden 6 des oberen Behältnisses 2 schichtförmig. Die Monozyten bzw. Makrophagen 5 liegen auf den Synoviozyten 4 und stehen mit diesen in direktem Kontakt. Die Synoviozyten 4 und die Monozyten bzw. Makrophagen 5 stammen aus Zelllinien humanen Ursprungs. Der Boden 6 des oberen Behältnisses 2 ist für zellulär sezernierte Stoffe durchlässig. Auf diese Weise ist ein Stoffaustausch zwischen dem oberen Behältnis 2 und dem unteren Behältnis 3 möglich. Das untere Behältnis 3 weist eine Vollblutkultur humanen Ursprungs mit Blutzellen 7 und 8 auf. Bei den Blutzellen handelt es sich insbesondere um Monozyten bzw. Makrophagen 5, Lymphozyten 7 und Erythrozyten 8. Die Vollblutkultur ist in Überstand und Sediment getrennt. Die im Zellkultursystem 1 anwesenden Zellen sowie die zwischen den Zellen ablaufenden Stoffflüsse ermöglichen eine verbesserte Simulation der entsprechenden zellulären Vorgänge im menschlichen Organismus. Daher kann das Zellkultursystem 1 in besonders geeigneter Weise für eine präklinische Untersuchung von Wirkstoffen herangezogen werden. Die hieraus gewonnenen Daten bzw. Informationen, insbesondere hinsichtlich einer medizinischen Hauptwirkung, etwaiger Nebenwirkungen sowie einer geeigneten Dosierung der Wirkstoffe, stellen eine verlässliche Beurteilungsgrundlage für die klinische Untersuchung der Wirkstoffe dar.

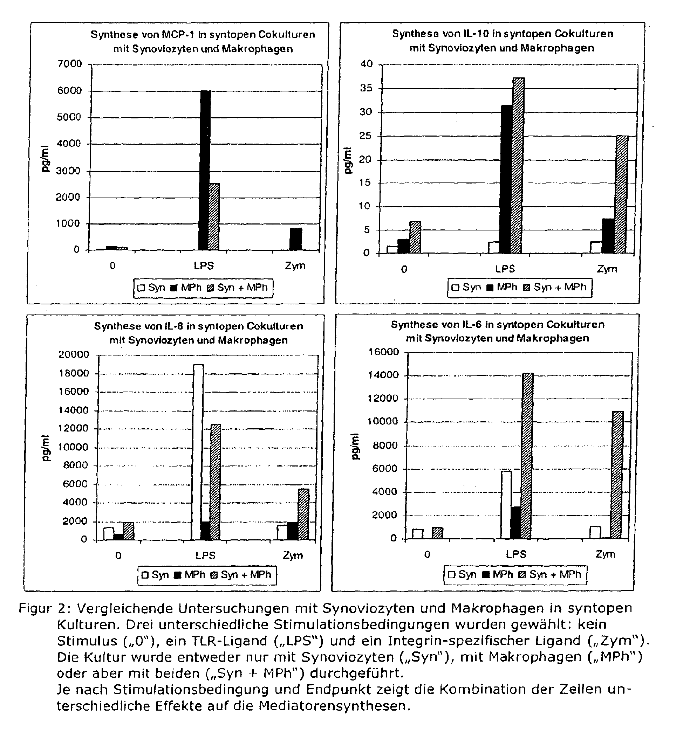

Figur 2 zeigt den Einfluss von verschiedenen Stimulanzien auf die Synthese von Botenstoffen bzw. Mediatoren sowohl bei klassischen Co-Kultursystemen als auch bei dem beschriebenen Zellkultursystem. Bei den klassischen Co-Kultursystemen handelte es sich um Kultursysteme, welche in ihrem oberen Kompartiment entweder eine Kultur mit Synoviozyten ("Syn") oder eine Kultur mit Makrophagen ("MPh") und in ihrem unteren Kompartiment eine Kultur des Vollblutes (Vollblutkultur) enthielten. Das hier beschriebene Zellkultursystem enthielt in seinem oberen Kompartiment eine syntope Kultur aus Synoviozyten und Makrophagen ("Syn + MPh") und in seinem unteren Kompartiment eine Kultur des Vollbluts (Vollblutkultur). Die Kultursysteme wurden drei unterschiedlichen Stimulationsbedingungen ausgesetzt:
- Keine Stimulierung ("0")
- Stimulierung mit einem Lipopolysaccharid ("LPS")
- Stimulierung mit Zymosan ("Zym").

Auf der Ordinate der Figuren 2a bis 2d ist jeweils die Menge des untersuchten synthetisierten Botenstoffes in Picogramm pro Milliliter [pg/ml] wiedergegeben. Auf der Abszisse der Figuren 2a bis 2d sind die Stimulationsbedingungen aufgeführt.

Die Figuren 2a bis 2d zeigen, dass abhängig von den Stimulationsbedingungen, den gemessenen Endpunkten (Synthese von MCP-1 im Falle von Figur 2a, Synthese von IL-10 im Falle von Figur 2b, Synthese von IL-8 im Falle von Figur 2c und Synthese von IL-6 im Falle von Figur 2d) sowie den verwendeten Kultursystemen unterschiedliche Mengen von Botenstoffen synthetisiert wurden.

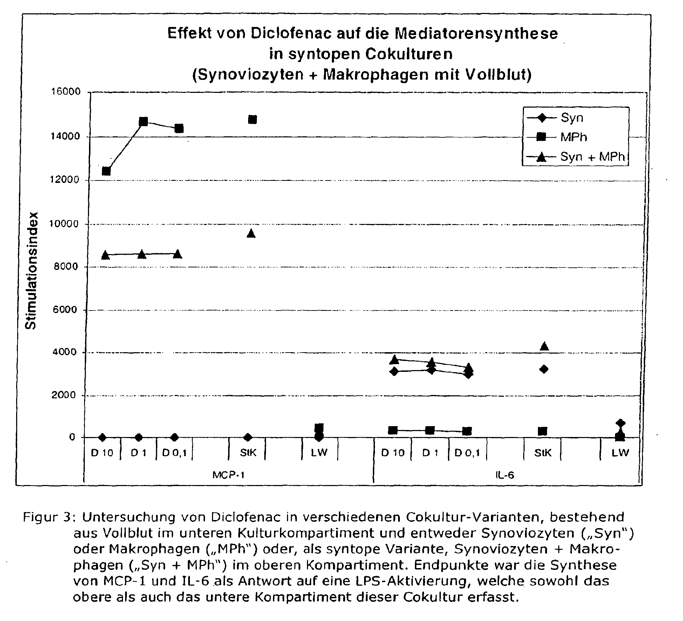

Figur 3 zeigt den Einfluss von Diclofenac (nicht steroidales Analgetikum) auf die Botenstoff- bzw. Mediatorensynthese bei klassischen Co-Kultursystemen und einen hier beschriebenen Zellkultursystem. Bezüglich der Merkmale der klassischen Co-Kultursysteme und des hier beschriebenen Zellkultursystems wird auf die Figurenbeschreibung zu Figur 2 verwiesen. Als Endpunkte wurde die Synthese von MCP-1 und IL-6 gemessen. Als Stimulationsbedingung wurde eine Aktivierung der Zellen durch ein Lipopolysaccharid gewählt. Auf der Ordinate von Figur 3 ist der sogenannte Stimulationsindex wiedergegeben. Auf der Abszisse von Figur 3 sind die gemessenen Endpunkte aufgeführt.

Die in den Figuren 2 und 3 wiedergegebenen Ergebnisse wurden mittels einer multiplexen Analyse auf der Basis eines sogenannten Bead-Array-Tests mit Hilfe der Luminex(TM)-Technologie durchgeführt. Hierfür wurden Farb-codierte Beads mit spezifischen Antikörper zum Binden der Botenstoffe eingesetzt. Der Gehalt an Botenstoffen wurde mit Hilfe eines zweiten Antikörpers gemessen, welcher fluoreszenzmarkiert war.

Die Offenbarung betrifft insbesondere auch die folgenden Aspekte:
1. Zellkultursystem, insbesondere zur präklinischen Erprobung von Wirkstoffen, umfassend ein erstes und ein zweites Kompartiment, welche über eine für zellulär sezernierte Stoffe durchlässige Trennschicht zwischen dem ersten und dem zweiten Kompartiment miteinander in Kommunikation stehen, wobei das erste Kompartiment eine syntope Kultur mit Gewebezellen und Immunzellen und das zweite Kompartiment eine Kultur mit Blutzellen aufweist.
2. Verfahren nach Aspekt 1, dadurch gekennzeichnet, dass es sich bei den Immunzellen um phagozytierende Immunzellen, insbesondere um Monozyten und/oder Makrophagen, handelt.
3. Zellkultursystem nach Aspekt 1 oder 2, dadurch gekennzeichnet, dass es sich bei den Gewebezellen um Zellen handelt, welche in entzündlich erkrankenden Geweben vorkommen.
4. Zellkultursystem nach einem der vorhergehenden Aspekte , dadurch gekennzeichnet, dass es sich bei den Gewebezellen um Epithelzellen und/oder um epithelähnliche Zellen handelt.
5. Zellkultursystem nach einem der vorhergehenden Aspekte dadurch gekennzeichnet, dass es sich bei den Gewebezellen um Bronchial- und/oder Darmepithelzellen handelt.
6. Zellkultursystem nach einem der vorhergehenden Aspekte dadurch gekennzeichnet, dass es sich bei den Gewebezellen um Endothelzellen, vorzugsweise um Blutgefäßendothetzellen, handelt.

## Patentansprüche

1. Kit zur präklinischen Erprobung von Wirkstoffen, mindestens umfassend ein erstes Kompartiment, welches eine syntope Kultur mit Gewebezellen und phagozytierende Immunzellen aufweist, und ein zweites Kompartiment mit einem Kulturmedium für Blutzellen,
**dadurch gekennzeichnet, dass** das erste und das zweite Kompartiment über eine für zellulär sezernierte Stoffe durchlässige Trennschicht zwischen dem ersten und dem zweiten Kompartiment miteinander in Kommunikation stehen, wobei das erste Kompartiment eine syntope Kultur mit Gewebezellen und phagozytierenden Immunzellen und das zweite Kompartiment eine Kultur mit Blutzellen aufweist.

2. Kit nach Anspruch 1, **dadurch gekennzeichnet, dass** das Kulturmedium eine Kulturflüssigkeit ist, beispielsweise eine Kulturlösung.

3. Kit nach Anspruch 1 oder 2, **dadurch gekennzeichnet, dass** es sich bei der Kultur des zweiten Kompartiments um eine Kultur des Vollbluts (Vollblutkultur), insbesondere von Frischblut, vorzugsweise humanen Ursprungs, handelt.

4. Kit nach einem der Ansprüche 1 bis 3, **dadurch gekennzeichnet, dass** die Trennschicht Öffnungen, insbesondere Poren, mit einem Durchmesser zwischen 0,1 und 5 µm, insbesondere zwischen 0,2 und 0,45 µm, aufweist.

5. Kit nach einem der Ansprüche 1 bis 4, **dadurch gekennzeichnet, dass** es sich bei den zellulär sezernierten Stoffen um Indikatoren zellulärer Aktivität, insbesondere um Botenstoffe, vorzugsweise um Zytokine, handelt.

6. Kit nach einem der vorhergehenden Ansprüche, **dadurch gekennzeichnet, dass** die Kompartimente als Behältnisse, insbesondere als Näpfe, Kammern oder Vertiefungen (Wells), ausgebildet sind.

7. Kit nach einem der vorhergehenden Ansprüche, **dadurch gekennzeichnet, dass** es sich bei den Immunzellen um Monozyten und/oder Makrophagen, handelt.

8. Kit nach einem der vorhergehenden Ansprüche, **dadurch gekennzeichnet, dass** es sich bei den Gewebezellen um Zellen handelt, welche in entzündlich erkrankenden Geweben vorkommen.

9. Kit nach einem der vorhergehenden Ansprüche, **dadurch gekennzeichnet, dass** es sich bei den Gewebezellen um Zellen handelt, die ausgewählt sind aus:
- Epithelzellen und/oder epithelähnliche Zellen,
- Bronchialzellen und/oder Darmepithelzellen,
- Endothelzellen, vorzugsweise Blutgefäßendothelzellen,
- Hautzellen, insbesondere Gelenkhautzellen (Synoviozyten), und/oder
- Chondrozyten.

10. Kit nach einem der vorhergehenden Ansprüche, **dadurch gekennzeichnet, dass** die Zellen aus einem der folgenden stammen:
- Zelllinien, vorzugsweise humanen Ursprungs,
- Gewebeproben und/oder Proben von Körperflüssigkeiten.

11. Kit nach einem der vorhergehenden Ansprüche, **dadurch gekennzeichnet, dass** die Gewebezellen aktiviert, insbesondere entzündlich verändert, sind.

12. Kit nach einem der vorhergehenden Ansprüche, **dadurch gekennzeichnet, dass** es sich bei den zu erprobenden Wirkstoffen um biologische und/oder synthetische Wirkstoffe handelt.

13. Kit nach einem der vorhergehenden Ansprüche, **dadurch gekennzeichnet, dass** er ein Blutentnahmebesteck umfasst.

14. Verwendung eines Kits nach einem der Ansprüche 1 bis 13 zur präklinischen Erprobung von Wirkstoffen, insbesondere zur Untersuchung von Dosis-Wirkungs-Beziehungen der Wirkstoffe.

## Claims

1. Kit for the pre-clinical testing of active substances, comprising at least a first compartment which has a syntopic culture with tissue cells and phagocytic immune cells, and a second compartment with a culture media for blood cells **characterised by** the first and second compartment being in communication with each other via a separating layer permeable for cellular secreted substances between the first and the second compartment, whereby the first compartment has a syntopic culture with tissue cells and phagocytic immune cells and the second compartment has a culture with blood cells.

2. Kit according to Claim 1, **characterised by** the culture medium being a culture fluid, such as a culture solution.

3. Kit according to Claim 1 or 2, **characterised by** the fact that the culture of the second compartment is a culture of whole blood (whole blood culture), especially of fresh blood, primarily of human origin.

4. Kit according to one of Claims 1 to 3, **characterised by** the fact that the separating layer has openings, in particular pores, with a diameter between 0.1 and 5 µm, in particular between 0.2 and 0.45 µm.

5. Kit according to one of the Claims 1 to 4, **characterised by** the fact that the cellular secreted substances are indicators of cellular activity, in particular chemical messengers, primarily cytokines.

6. Kit according to one of the preceding Claims, **characterised by** the fact that the compartments are formed as receptacles, in particular as dishes, chambers or wells.

7. Kit according to one of the preceding Claims, **characterised by** the fact that the immune cells are monocytes and/or macrophages.

8. Kit according to one of the preceding Claims, **characterised by** the fact that the tissue cells are cells which occur in inflamed tissues.

9. Kit according to one of the preceding Claims, **characterised by** the fact that the tissue cells are cells which are selected from:
- Epithelial cells and/epithelioid cells,
- Bronchial cells and/or enterocytes,
- Endothelial cells, primarily blood vessel endothelial cells,
- Skin cells, in particular joint skin cells (synoviocytes), and/or
- Chondrocytes.

10. Kit according to one of the preceding Claims, **characterised by** the fact that the cells originate from one of the following:
- Cell lines, primarily of human origin,
- Tissue samples and/or samples of bodily fluids.

11. Kit according to one of the preceding Claims, **characterised by** the fact that the tissue cells are activated, in particular that they display inflammatory changes.

12. Kit according to one of the preceding Claims, **characterised by** the fact that the active substances to be sampled are biological and/or synthetic active substances.

13. Kit according to one of the preceding Claims, **characterised by** the fact that it comprises blood-taking instruments.

14. The use of a kit according to one of the Claims 1 to 13 for the pre-clinical testing of active substances, in particular for testing the dose-effect relationships of the active substances.

## Revendications

1. Kit pour l'essai préclinique de principes actifs, comprenant au moins un premier compartiment qui contient une culture syntope comportant des cellules tissulaires et des cellules immunitaires phagocytaires, et un deuxième compartiment comportant un agent de culture pour les cellules sanguines, **caractérisé en ce que** le premier compartiment et le deuxième compartiment sont en communication ensemble par une couche de séparation qui est transparente pour des substances cellulaires sécrétées entre le premier compartiment et le deuxième compartiment, sachant que le premier compartiment contient une culture syntope comprenant des cellules tissulaires et des cellules immunitaires phagocytaires, et le deuxième compartiment une culture comprenant des cellules sanguines.

2. Kit selon la revendication 1, **caractérisé en ce que** le milieu de culture est un fluide de culture, par exemple une solution de culture.

3. Kit selon la revendication 1 ou 2, **caractérisé en ce que** la culture du deuxième compartiment est une culture du sang complet (culture de sang complet), en particulier de sang frais, de préférence d'origine humaine.

4. Kit selon l'une des revendications 1 à 3, **caractérisé en ce que** la couche de séparation comporte des ouvertures, en particulier des pores, d'un diamètre compris entre 0,1 et 5 µm, en particulier entre 0,2 et 0,45 µm.

5. Kit selon l'une des revendications 1 à 4, **caractérisé en ce que** les substances cellulaires sécrétées sont des indicateurs d'activité cellulaire, en particulier des substances messagères, de préférence des cytokines.

6. Kit selon l'une des revendications qui précèdent, **caractérisé en ce que** les compartiments sont confgurés en tant que récipients, en particulier jattes, chambres ou cavité (« wells » ou puits).

7. Kit selon l'une des revendications qui précèdent, **caractérisé en ce que** les cellules immunitaires sont des monocytes et/ou des macrophages.

8. Kit selon l'une des revendications qui précèdent, **caractérisé en ce que** les cellules tissulaires sont des cellules qui sont présentes dans des tissus qui sont à l'origine de maladies inflammatoires.

9. Kit selon l'une des revendications qui précèdent, **caractérisé en ce que** les cellules tissulaires sont des cellules qui sont sélectionnées parmi les éléments suivants :
- cellules épithéliales et/ou cellules apparentées aux cellules épithéliales,
- cellules bronchiques et/ou cellules épithéliales de l'intestin,
- cellules endothéliales, de préférence cellules épithéliales des vaisseaux sanguins,
- cellules cutanées, en particulier cellules cutanées des articulations (synoviocytes), et/ou
- chondrocytes.

10. Kit selon l'une des revendications qui précèdent, **caractérisé en ce que** les cellules proviennent de l'une des sources suivantes :
- lignées cellulaires, de préférence d'origine humaine,
- échantillons de tissu et/ou prélèvements de fluides corporels.

11. Kit selon l'une des revendications qui précèdent, **caractérisé en ce que** les cellules tissulaires sont activées, et en particulier modifiées par une inflammation.

12. Kit selon l'une des revendications qui précèdent, **caractérisé en ce que** les principes actifs à tester sont des principes actifs biologiques et/ou synthétiques.

13. Kit selon l'une des revendications qui précèdent, **caractérisé en ce qu'**il comprend un outil de prélèvement sanguin.

14. Utilisation d'un kit selon l'une des revendications 1 à 13 pour des essais précliniques de principes actifs, en particulier pour l'analyse des relations entre dose et effet des principes actifs.
